# EUROPEAN PATENT APPLICATION

(11) **EP 4 744 595 A1**
(43) Date of publication of application: **20.05.2026**
(21) Application number: 24212898.1
(22) Date of filing: 14.11.2024
(51) Int. Cl.: A61B 6/46, A61B 6/50

(54) **X-RAY IMAGING SYSTEM AND METHOD**

(71) Applicant: Koninklijke Philips N.V., 5656 AE Eindhoven (NL)
(72) Inventor: GRASS, Michael, Eindhoven (NL); KLINDER, Tobias, Eindhoven (NL); KOEHLER, Thomas, 5656AG Eindhoven (NL)
(74) Representative: Philips Intellectual Property & Standards

(57) **Abstract**

An X-ray imaging system generates a first image comprising an attenuation image and at least a second image of a different type. An image is generated for display comprising the first image. An analysis is performed of the first image and/or the second image to detect image characteristics which warrant further investigation by the user of the imaging system. An image overlay is the applied to the image for display if said image characteristics are detected that warrant said further investigation. In this way, the user is only presented with multi-image information when it is suitable to do so.

## Description

### FIELD OF THE INVENTION

This invention relates to X-ray imaging systems having multiple imaging modalities.

### BACKGROUND OF THE INVENTION

One example of an X-ray imaging system which can generate multiple image types is dark-field X-ray imaging. Dark-field imaging has attracted much interest, especially in the medical field. This imaging system is based on the acquisition of attenuation and wave properties that X-rays experience when travelling through the human body. Acquisitions are performed with a modified X-ray system using pre- and post-patient gratings.

Dark-field X-ray imaging quantifies the small-angle scattering that is occurring in the object due to differences in the refractive index of different materials.

Differential phase imaging detects phase gradients differentially across various regions of the image. It provides more detailed information about the gradients in refractive index or other material properties by measuring how the phase shifts change from point to point.

Dark-field and differential phase images can be generated by the same dark-field X-ray imaging system. The acquisition of an X-ray dark-field image and a differential phase image relies on a three grating interferometer, that is used to differentiate between the attenuation of radiation, small-angle scattering and refraction. Thus, imaging with an interferometer provides three independent images: conventional attenuation, dark-field and phase-contrast. The three imaging modalities are intrinsically perfectly registered. The information provided by the X-ray dark-field image and the differential phase image can be used for diagnostic purposes for example to enhance a diagnosis from an attenuation image alone.

Particularly for pulmonary imaging, X-ray dark-field imaging, but also phase-contrast imaging, has been identified as a possibility to significantly increase the diagnostic sensitivity and specificity.

While the quantity of information that can be generated using a dark-field X-ray system is of great benefit, reading X-ray images is very time-critical and increasing the amount of available information via additional images may not be advantageous in the radiological workflow.

Another example is a spectral X-ray detector system. A spectral X-ray detector system (also known as an energy-resolving X-ray detector) is an X-ray detection technology that not only measures the intensity of X-rays passing through a sample but also provides information about the energy of individual X-ray photons. This energy resolution capability allows for better material characterization, tissue differentiation, and reduced noise in X-ray imaging systems.

Spectral X-ray detectors are for example formed as dual layer or triple layer detection systems. The energy information obtained by spectral X-ray detectors allow the system to create multiple energy "bins" or channels, where each bin corresponds to a specific range of energies. By using these bins, the system can generate different images for different energy ranges, enabling material decomposition and better differentiation of structures. Thus, a spectral X-ray detector can generate at least two types of image; a conventional attenuation image and various additional a spectral (energy-resolved) images.

There is a need to simplify the analysis of X-ray images, when there are multiple imaging modalities.

### SUMMARY OF THE INVENTION

The invention is defined by the claims.

According to examples in accordance with an aspect of the invention, there is provided an X-ray imaging system, comprising:
an X ray source;
an X-ray detector;
a processing system for processing the signals received by the X-ray detector and for generating X-ray images, wherein the processing system is configured to generate a first image comprising an attenuation image and at least a second image of a different type; and
a display,
wherein the processing system is configured to:
   generate an image for display comprising the first image;
   perform an analysis of the first image and/or the second image to detect image characteristics which warrant further investigation by the user of the imaging system; and
   apply an image overlay to the image for display if said image characteristics are detected that warrant said further investigation, wherein the image overlay relates to the second image.

This imaging system presents by default an attenuation (first) image to the user when performing an examination using the X-ray imaging system. However, at least second images are also generated but they are not displayed by default. Instead, an automated alerting system is used to analyze some or all of the images. For example, all acquired images may be analyzed including the conventional attenuation image.

An alert is triggered when image characteristics are found that warrant further investigation. By this is meant that the user is recommended to view additional information to that present in the first image. As discussed below, different rules may be used to determine when image characteristics warrant further investigation.

The system addresses the workflow challenge radiologists are confronted with when implementing a multimodal X-ray imaging in clinical practice, for example dark-field X-ray imaging. As mentioned above, a dark-field X-ray imaging system generates up to three different images compared to a conventional image.

The presentation of additional information to that of the attenuation image is limited by this system when performing an examination of a subject. Once an alert is activated, information relating to the second image is displayed. This may be information from the second image, or it may be an indication that the second image should be consulted or it may be a measurement obtained using the second image. Thus, the system decides for the user which images it might be worth inspecting instead of a default workflow where all the available information is read and displayed to the user.

Specific diseases to detect that benefit from an analysis of the second image are for example chronic obstructive pulmonary disease (COPD), pulmonary fibrosis, bronchopulmonary dysplasia (BPD), cancer, pneumothorax, acute pulmonary inflammation, and lymphangioleiomyomatosis (LAM).

The image overlay for example comprises a recommendation to consult the second image. Thus, the user has the choice of whether or not to view additional images.

The image overlay for example comprises a portion of the second image. In this way, relevant parts of the second image can be displayed as an overlay when the analysis shows the second image to be relevant.

The imaging system may further comprise a pointer to enable the user of the system to identify a portion of interest of the first image, wherein the processing system is configured to apply the image overlay when the user of the system identifies a portion of the first image, and the portion of the at least second image corresponds to the portion of the first image.

Thus, the portion of the second image is only presented to the user when the user is pointing to (hence looking at) parts of the first image for which the corresponding part of the second image will enhance the information content. In this case, the image characteristics that warrant further investigation are image regions pointed to by the user.

The image overlay may comprise an anatomical measurement obtained using the second image. For example, the image characteristics which warrant further investigation for example comprise image portions of the primary (attenuation) image from which user measurements have been made. Thus, when a user makes measurements using the primary image, this indicates that there are image characteristics which warrant investigation. The secondary image may then be used to confirm or correct the measurements obtained using the primary image. The overlay may then comprise anatomical measurements derived from the secondary image.

The image characteristics which warrant further investigation for example comprise disease detection characteristics. Thus, the user is guided to look at additional image information when it may be of assistance in making a disease diagnosis.

The image overlay may then comprise a disease identification. The system may perform automated disease identification so that the user can then inspect the second image (as well as the first) to confirm the automated disease diagnosis.

The processing system may comprise a trained AI network for image-based disease detection at least from images of the same type as the second image.

The processing system may be configured to perform an analysis of the first image and the at least second image to detect said image characteristics. The image characteristics (e.g. disease detection characteristics) may be assessed for both (or indeed all) generated images. The system can then determine if the second (or further) image provides additional relevant information to the default attenuation image and hence should be consulted by the user.

The second image for example comprises a dark-field image or a differential phase image or a photoelectric effect image or a Compton image or a k-edge image or a spectral image. Thus, the invention may be applied to various X-ray systems that generate at least one image using a different imaging modality to a basic attenuation image.

The image processing system may be configured to generate a first image comprising an attenuation X-ray image, a second image comprising a dark-field image and a third image comprising a differential phase image, and the processing system is further configured to:
perform an analysis of the third image to detect image characteristics which warrant further investigation by the user of the imaging system,
wherein the image overlay relates to the third image.

Thus, there may be three images. The third image is treated in the same way as the second image, i.e. it is not displayed by default and is analyzed to detect image characteristics which warrant further investigation by the user of the imaging system.

The image overlay may then comprise an identification of whether the second or third image is the most suitable to consult in respect of the image characteristics which warrant further investigation. The system may thus choose what image information, additional to the attenuation image, is relevant for the user, and recommend which additional image should be consulted, or indeed present information from that additional image.

The image processing system may be configured to generate a first image comprising an attenuation X-ray image and a second image comprising a Compton image and/or a third image comprising a k-edge image. These images may be obtained by a photon counting X-ray detector, such as a triple layer detector.

The invention also provides an X-ray imaging method, comprising:
controlling an X-ray imaging system to generate an image for display comprising a first X-ray image that is an attenuation image and to generate a second image of a different type to the first image;
performing an analysis of the first image and/or the second image to detect image characteristics which warrant further investigation by the user of the X-ray imaging system; and
applying an image overlay to the image for display if said image characteristics are detected that warrant said further investigation, wherein the image overlay relates to the second image.

The image overlay for example comprises a recommendation to consult the second image or comprises a portion of the second image. The image characteristics which warrant further investigation for example comprise disease detection characteristics.

The invention also provides a computer program comprising computer program code which is adapted, when said program is run on the processing system of an X-ray imaging system, to perform the method above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an X-ray imaging system;
Fig. 2 shows an X-ray imaging method;
Fig. 3A shows a normal X-ray attenuation image of a healthy patient;
Fig. 3B shows a dark-field image of a healthy patient;
Fig. 3C shows a normal X-ray attenuation image of a diseased patient;
Fig. 3D shows a dark-field image of a diseased patient;
Fig. 4A shows the image of Fig. 3C with an alert overlayed on top in the form of an identification of an area of concern; and
Fig. 4B shows the display of dark-field information on top of the attenuation image in the area of concern.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an X-ray imaging system that generates a first image comprising an attenuation image and at least a second image of a different type. An image is generated for display comprising the first image. An analysis is performed of the first image or the second image or both images to detect image characteristics which warrant further investigation by the user of the imaging system. An image overlay is the applied to the image for display if said image characteristics are detected that warrant said further investigation. In this way, the user is only presented with multi-image information when it is suitable to do so.

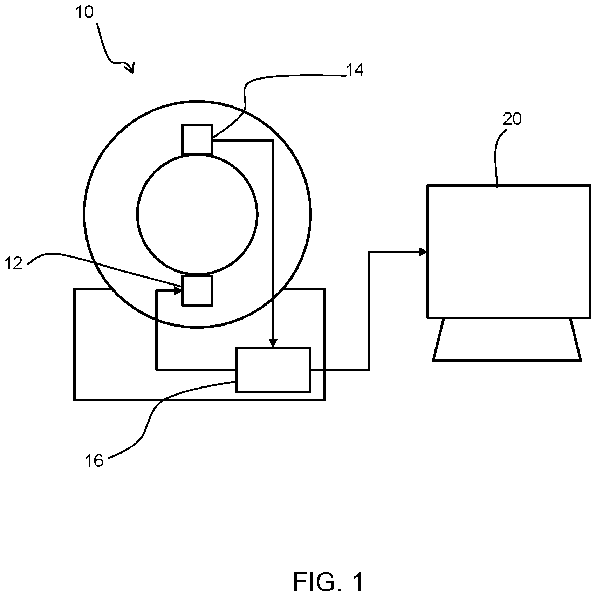

Fig. 1 shows an X-ray imaging system 10 comprising an X ray source 12, an X-ray detector 14 and a processing system 16 for processing the signals received by the X-ray detector 14 and for generating X-ray images.

The X-ray imaging system able to generate different image types, including at least an attenuation image. There are various types of multi-modal X-ray imaging systems, including dark-field X-ray imagers, photon counting X-ray images and X-ray imagers using triple layer detectors. Different image types have different diagnostic values for different disease detection functions.

For example, a dark-field X-ray detector may also enable various image types to be derived, such as:
attenuation images (traditional X-ray images);
dark-field images;
phase contrast images;
differential phase images;
tomosynthesis images.

A triple layer detector, or a photon counting detector (which may also use a triple layer detector), may enable various image types to be derived, such as:
attenuation images (traditional X-ray images);
Compton images;
spectral images;
k-edge Images;
a photoelectric effect image
a bone image; and
a soft tissue image.

The X-ray imaging system further comprises a display 20. The processing system 16 generates a first image comprising an attenuation image and at least a second image of a different type.

The different image type is generated from the same X-ray hardware, but it highlights different features and thus has different diagnostic benefits. The multiple images are registered with each other because they are obtained from the same source and detector configuration.

The processing system generates the image for display. It comprises the first (attenuation) image, but depending on the image content, that attenuation image is supplemented with information relating to the second imager. For this purpose, an analysis is conducted at least of the second image, but preferably of both the first and second images, to detect image characteristics which warrant further investigation by the user of the imaging system. If such image characteristics are detected. an image overlay is applied to the image for display. The image overlay may identify the second image (i.e. indicate to the user that they should choose to consult the second image) or it may even include a portion of the second image, to provide the user with an image region with improved diagnostic clarity.

Thus, the imaging system presents by default the attenuation image to the user. Second images are also generated but they are not displayed by default. Instead, an automated alerting system is used to indicate when the second images are of interest to the user.

The system addresses the workflow challenge radiologists are confronted with when implementing a multimodal X-ray imaging in clinical practice. Information overload is avoided, and replaced with an alert approach.

Specific diseases to detect that benefit from an analysis of a second image, in addition to an attenuation image, are for example chronic obstructive pulmonary disease (COPD), pulmonary fibrosis, bronchopulmonary dysplasia (BPD), cancer, pneumothorax, acute pulmonary inflammation, and lymphangioleiomyomatosis.

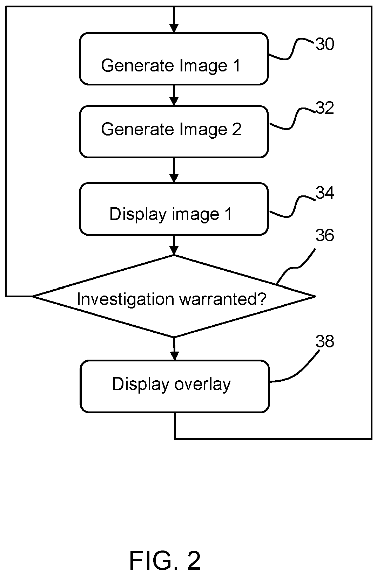

Fig. 2 shows an X-ray imaging method 30.

In step 32, the X-ray imaging system is controlled to generate an image for display comprising a first, attenuation X-ray image .

In step 34, a second image is generated of a different type.

In step 36 the first image is displayed.

In step 38, an analysis is performed the first image or the second image or both (and preferably of all images when there are three or more), to detect image characteristics which warrant further investigation by the user of the X-ray imaging system.

If no investigation is warranted, the next images are generated.

If an investigation is warranted, an image overlay is applied to the image for display in step 40. The image overlay relates to (i.e. identifies or copies a part of) the second image.

One example of imaging system is a dark-field X-ray system. In one implementation of such a system, in addition to generating an attenuation image, a dark-field image and a differential phase image may be generated. Although the additional information provided by a dark-field system is in general beneficial, it does not add significant value to each scan. Thus, deciding upfront for which scan it might be worth inspecting all available information is a significant aid, compared to a default workflow where the information is read every time. Thus, by having an automated algorithm it is ensured that all available information is consistently read, but only in cases where it really matters, the user will be notified.

The image characteristics which warrant further investigation, mentioned above, for example comprise disease detection characteristics. In other words, they are image characteristics which lead to a diagnosis that there is an increased likelihood of the presence of a particular disease. The image overlay may then identify the disease.

The automated disease diagnosis for example is made using an active neural network, wherein a trained AI network is used for disease detection based on one or more of the images, such as the dark-field image, the differential phase image or combinations of dark-field, differential phase, and attenuation methods.

When a disease is found, the alert may highlight to the user that the differential phase image and/or dark-field image should be inspected. The user can be guided to the relevant image for additional information.

When there are two or more additional images to the default attenuation image, the image overlay may highlight which additional image (e.g. the differential phase image or dark-field image) is of most relevance. To identify which image is important, different options are available, such as measuring the contrast between object and background in the different images and determining the one with the highest contrast, or analyzing the histogram of image values in the different images on variable scales.

Different AI models may be run on the various possible combinations of data (e.g., conventional-only, differential phase-only, dark-field-only, conventional plus differential phase, conventional plus dark-field, differential phase plus dark-field, conventional plus differential phase plus dark-field), and predictions can then be made.

The use of automated disease detection is on option. In a more basic implementation, the imaging system has a pointer to enable the user of the system to identify a portion of interest of the first image (i.e. a mouse). The processing system may then apply the image overlay when the user of the system identifies a portion of the first image. The portion of the second (or third etc.) image corresponds to the portion of the first image.

Thus, when moving the mouse over the default image, additional relevant information from the other images, such as the differential phase image or dark-field image is displayed on top of the conventional X-ray image. This does not increase the reading time compared to classical attenuation X-ray imaging.

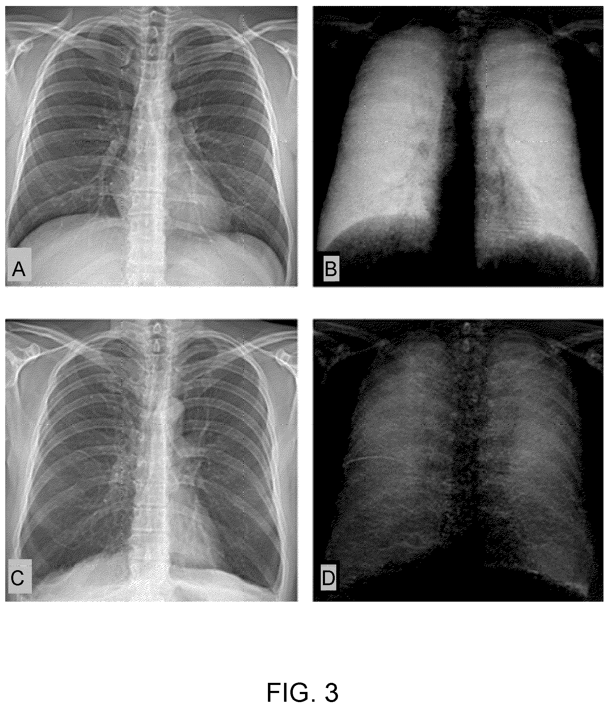

Fig. 3A shows a normal X-ray attenuation image and Fig. 3B shows a dark-field image, each of a healthy patient.

Fig. 3C shows a normal X-ray attenuation image and Fig. 3D shows a dark-field image, each of a diseased patient. These images are taken from Zimmermann et al., Eur. Rad. Exp. 6, 2022.

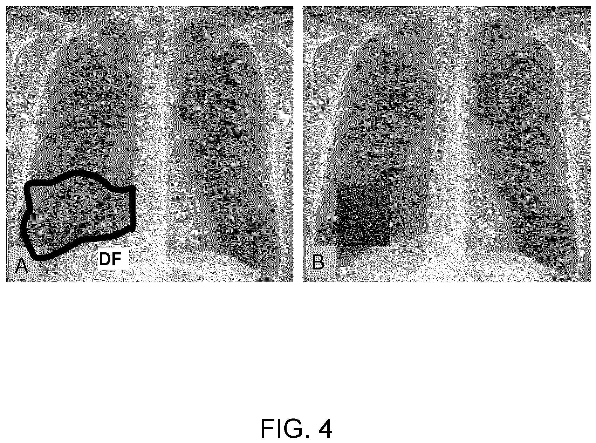

Fig. 4A shows the image of Figure 3C with an alert overlayed on top in the form of an identification of the area of concern and an indication that the dark-field image should be consulted. Fig. 4B shows the display of dark-field information on top of the attenuation image in the area of concern. Thus, Fig. 4 shows two possible examples of overlay information over the conventional attenuation image.

The image characteristics which warrant further investigation may be defined by the user, such as predefined combinations of image values in the conventional attenuation image and in the second image, e.g. in a dark-field image. These may be determined in regions of interest with a variable scale. Histogram distributions may be analyzed with variable scale.

Another possible feature is that if a radiologist performs an image or disease or anatomy quantification by segmentation or measurement on the conventional image, automatic feedback may be generated on the accuracy from the additional images. Thus, in this case, the image characteristics which warrant further investigation are image features that have been measured by the user. The overlay then comprises corresponding measurements that have been made automatically using the other image or images.

Measurements may be obtained automatically from the second image which indicate that the second image should be inspected. The image overlay may then indicate that the second image should be consulted.

Thus, the image overlay may comprise one or more of
a portion of the second image;
an indication that the second image should be consulted;
a measurement obtained from the second image.

The image overlay may be triggered by:
the results of automatic measurements made to the first image;
the results of automatic measurements made to the second image;
anatomical measurements having been made by the user from the first image;
the user highlighting areas of interest of the first image;
a disease diagnosis from the first image.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An X-ray imaging system (10), comprising:
an X ray source (12);
an X-ray detector (14);
a processing system (16) for processing the signals received by the X-ray detector and for generating X-ray images, wherein the processing system is configured to generate a first image comprising an attenuation image and at least a second image of a different type to the first image; and
a display (20),
wherein the processing system (16) is configured to:
generate an image for display comprising the first image;
perform an analysis of the first image and/or the second image to detect image characteristics which warrant further investigation by the user of the imaging system; and
apply an image overlay to the image for display if said image characteristics are detected that warrant said further investigation, wherein the image overlay relates to the second image.

2. The imaging system of claim 1, wherein the image overlay comprises a recommendation to consult the second image.

3. The imaging system of claim 1, wherein the image overlay comprises a portion of the second image.

4. The imaging system of claim 3, further comprising a pointer system to enable the user of the system to identify a portion of interest of the first image, wherein the processing system is configured to apply the image overlay when the user of the system identifies a portion of the first image, and the portion of the second image corresponds to the portion of the first image.

5. The imaging system of claim 1, wherein the image overlay comprises an anatomical measurement obtained using the second image.

6. The imaging system of any one of claims 1 to 5, wherein the image characteristics which warrant further investigation comprise disease detection characteristics and wherein the image overlay comprises a disease identification.

7. The imaging system of claim 6, wherein the processing system comprises a trained AI network for image-based disease detection at least from images of the same type as the second image.

8. The imaging system of any one of claim 1 to 7, wherein the processing system is configured to perform an analysis of the first image and the at least second image to detect said image characteristics.

9. The imaging system of any one of claims 1 to 8, wherein the second image comprises a dark-field image or a differential phase image or a photoelectric effect image or a Compton image or a k-edge image or a spectral image.

10. The imaging system of any one of claims 1 to 9, wherein the image processing system is configured to generate a first image comprising an attenuation X-ray image, a second image comprising a dark-field image and a third image comprising a differential phase image, and wherein the processing system is further configured to:
perform an analysis of the third image to detect image characteristics which warrant further investigation by the user of the imaging system,
wherein the image overlay relates to the third image.

11. The imaging system of claim 10, wherein the image overlay comprises an identification of whether the second or third image is the most suitable to consult in respect of the image characteristics which warrant further investigation.

12. The imaging system of any one of claims 1 to 9, wherein the image processing system is configured to generate a first image comprising an attenuation X-ray image, a second image comprising a Compton image and optionally a third image comprise a k-edge image.

13. An X-ray imaging method, comprising:
(30) controlling an X-ray imaging system to generate an image for display comprising a first X-ray image that is an attenuation image and (32) to generate a second image of a different type to the first image;
(36) performing an analysis of the first image and/or the second image to detect image characteristics which warrant further investigation by the user of the X-ray imaging system; and
(38) applying an image overlay to the image for display if said image characteristics are detected that warrant said further investigation, wherein the image overlay relates to the second image.

14. The method of claim 13, wherein the image overlay comprises a recommendation to consult the second image or comprises a portion of the second image and wherein the image characteristics which warrant further investigation comprise disease detection characteristics.

15. A computer program comprising computer program code which is adapted, when said program is run on the processing system of an X-ray imaging system, to perform the method of claim 13.
